# EUROPEAN PATENT APPLICATION

(11) **EP 4 295 858 A1**
(43) Date of publication of application: **27.12.2023**
(21) Application number: 22213287.0
(22) Date of filing: 24.08.2012
(51) Int. Cl.: A61K 38/22, A61M 31/00, A61K 38/28, A61K 9/00, A61K 38/17, A61K 38/26

(54) **FORMULATIONS OF ACTIVE AGENTS FOR SUSTAINED RELEASE**

(30) Priority: 24.08.2011 US 201161526940 P; 26.10.2011 US 201161551506 P
(62) Divisional of application: 22181022.9
(71) Applicant: ImmunoForge Co., Ltd., Gasan digital 1-ro, Geumcheon-gu Seoul 08591 (KR)
(72) Inventor: ARNOLD, Susan, Malvern, Pennsylvania (US); PRIOR, Christopher, Malvern, Pennsylvania (US)
(74) Representative: Cooley (UK) LLP

(57) **Abstract**

The present invention provides pharmaceutical formulations for sustained release, and methods for delivering a treatment regimen with a combination of sustained release and long half-life formulations. The invention provides improved pharmacokinetics for peptide and small molecule drugs. A sustained release pharmaceutical formulation comprises a therapeutic agent for systemic administration, where the therapeutic agent comprises an active agent and an amino acid sequence capable of forming a reversible matrix at the body temperature of the subject.

## Description

### PRIORITY

This application claims priority to U.S. Provisional Application No. 61/526,940, filed August 24, 2011 and U.S. Provisional Application No. 61/551,506, filed October 26, 2011, each of which are hereby incorporated by reference in its entirety.

### FIELD OF INVENTION

The present invention relates to pharmaceutical formulations for sustained release, and methods for delivering a treatment regimen with the sustained release formulations.

### DESCRIPTION OF THE TEXT FILE SUBMITTED ELECTRONICALLY

The contents of the text file submitted electronically herewith are incorporated herein by reference in their entirety: a computer readable format copy of the sequence listing (filename: PHAS_024_02WO_SeqList_ST25.txt, date recorded: August 14, 2012, file size 3 kilobytes).

### BACKGROUND

The effectiveness of peptide and small molecule drugs is often limited by the half-life of such drugs in the circulation, as well as difficulties in obtaining substantially constant plasma levels. For example, the incretin GLP-1 must be administered at relatively high doses to counter its short half-life in the circulation, and these high doses are associated with nausea, among other things. Murphy and Bloom, Nonpeptidic glucagon-like peptide 1 receptor agonists: A magic bullet for diabetes? PNAS 104 (3):689-690 (2007). Further, the peptide agent vasoactive intestinal peptide (VIP) exhibits a half-life, in some estimates, of less than one minute, making this agent impractical for pharmaceutical use. Domschke et al., Vasoactive intestinal peptide in man: pharmacokinetics, metabolic and circulatory effects, Gut 19:1049-1053 (1978); Henning and Sawmiller, Vasoactive intestinal peptide: cardiovascular effects, Cardiovascular Research 49:27-37 (2001). A short plasma half life for peptide drugs is often due to fast renal clearance as well as to enzymatic degradation during systemic circulation.

Strategies for improving the pharmacokinetics of peptide and small molecule drugs are in great demand.

### SUMMARY OF THE INVENTION

The present invention provides pharmaceutical formulations for sustained release, and methods for delivering a treatment regimen with the sustained release formulations. The invention thereby provides improved pharmacokinetics for peptide and small molecule drugs.

In one aspect, the invention provides a sustained release pharmaceutical formulation. The formulation comprises a therapeutic agent for systemic administration, where the therapeutic agent comprises an active agent and an amino acid sequence capable of forming a reversible matrix at the body temperature of a subject. The reversible matrix is formed from hydrogen bonds (e.g., intra- and/or intermolecular hydrogen bonds) as well as from hydrophobic contributions. The formulation further comprises one or more pharmaceutically acceptable excipients and/or diluents inducing the formation of the matrix upon administration. The matrix provides for a slow absorption to the circulation from an injection site. The sustained release, or slow absorption from the injection site, is due to a slow reversal of the matrix as the concentration dissipates at the injection site. Once product moves into the circulation, the formulation confers long half-life and improved stability. Thus, a unique combination of slow absorption and long half-life is achieved leading to a desirable PK profile with a shallow peak to trough ratio and/or long Tmax.

In certain embodiments, the amino acid sequence is an Elastin-Like-Protein (ELP) sequence. The ELP sequence comprises or consists of structural peptide units or sequences that are related to, or mimics of, the elastin protein. The amino acid sequence may exhibit a visible and reversible inverse phase transition with the selected formulation. That is, the amino acid sequence may be structurally disordered and highly soluble in the formulation below a transition temperature (Tt), but exhibit a sharp (2-3°C range) disorder-to-order phase transition when the temperature of the formulation is raised above the Tt. In addition to temperature, length of the amino acid polymer, amino acid composition, ionic strength, pH, pressure, selected solvents, presence of organic solutes, temperature, and protein concentration may also affect the transition properties, and these may be tailored for the desired absorption profile. Other exemplary sequences or structures for the amino acid sequence forming the matrix are disclosed herein.

In various embodiments, the active agent for systemic administration is a protein or peptide, which may have a short circulatory half-life, such as from about 30 seconds to about 1 hour, to about 2 hours, or to about 5 hours. In some embodiments, the protein or peptide has a circulatory half-life of from 30 seconds to about 10 hours. The therapeutic agent may be a recombinant fusion protein between the protein active agent and the amino acid sequence capable of forming the matrix. Exemplary peptide active agents include GLP-1 receptor agonists (e.g., GLP-1 or derivative thereof), glucagon receptor agonists (*e.g.* glucagon, oxyntomodulin or derivatives thereof), VPAC2 selective agonists (*e.g.* vasoactive intestinal peptide (VIP) or a derivative thereof), GIP receptor agonists (*e.g.* glucose-dependent insulinotropic peptide (GIP) or a derivative thereof) or insulin or a derivative thereof. Alternatively, the protein active agent is a clotting factor, such as Factor VII, Factor VIII, or Factor IX. Other protein and small molecule drugs for delivery in accordance with the invention are disclosed herein. By providing a slow absorption from the injection site, renal clearance and degradation can be controlled thereby achieving the desired PK profile.

In another aspect, the invention provides a method for delivering a sustained release regimen of an active agent. The method comprises administering the formulation described herein to a subject in need, wherein the formulation is administered from about 1 to about 8 times per month. In some embodiments, the formulation is administered about weekly, and may be administered subcutaneously or intramuscularly (for example). In some embodiments, the site of administration is not a pathological site, that is, the therapeutic agent is not administered directly to the intended site of action.

### DESCRIPTION OF THE DRAWINGS

**Figure 1** shows the phase transition (as shown by an increase in turbidity) of an ELP1 protein, induced by a change in temperature to 37°C or above. This property provides for a slow absorption from an injection site.
**Figure 2** shows the phase transition (as shown by an increase in turbidity) of an ELP4 protein, induced by a change in temperature to 25°C or above. This property provides for a depot-like delivery.
**Figure 3** illustrates, without wishing to be bound by theory, a potential mechanism for the observed transition, in which a water shell is excluded under certain conditions, allowing for hydrogen bonds to form.
**Figure 4** shows that the ELP4 series transitions at 37°C at a protein concentration of less than about 0.01 mg/ml, allowing for sustained release formulations of low protein concentration, for example, at the injection site.
**Figure 5** shows that the ELP1 series transitions at just below 37°C at relatively high protein concentration of about 10 mg/ml or more, allowing for sustained release formulations with relatively high amounts of active agents.
**Figure 6** shows a summary of pharmacokinetic parameters for Glp-1/ELP1-120 (also referred to herein as PB1023 or Glymera) after SC administration of 0.3, 0.6, 0.9 and 1.35 mg/kg to adult subjects with type 2 diabetes mellitus.
**Figure 7** shows the mean serum concentrations of Glp-1/ELP1-120 (also referred to herein as PB1023 or Glymera) after s.c. administration on day 0 of 0.3, 0.6, 0.9 and 1.35 mg/kg to adult subjects with type 2 diabetes mellitus (semi-logarithmic axes).
**Figure 8** shows the type 2 diabetes mellitus: Glymera program overview pharmacokinetics crossover study. Mean serum concentrations of Glymera following s.c. administration of 90 mg as 50 mg/mL and 100 mg/mL formulations to adult subjects with type 2diabetes mellitus are shown (semi-logarithmic axes).
**Figure 9** shows a summary of pharmacokinetic parameters for Glymera after s.c. administration of 90 mg as 50 mg/mL and 100 mg/mL formulations to adult subjects with type 2 diabetes mellitus.

### DETAILED DESCRIPTION

The present invention provides pharmaceutical formulations for sustained release, and methods for delivering a treatment regimen with the sustained release formulations. The invention thereby provides improved pharmacokinetics for peptide and small molecule drugs, including a relatively flat PK profile with a low ratio of peak to trough, and/or a long Tmax. The PK profile can be maintained with a relatively infrequent administration schedule, such as from one to eight injections per month in some embodiments.

In one aspect, the invention provides a sustained release pharmaceutical formulation. The formulation comprises a therapeutic agent for systemic administration, where the therapeutic agent comprises an active agent and an amino acid sequence capable of forming a matrix at the body temperature of a subject. The reversible matrix is formed from hydrogen bonds (*e.g.,* intra- and/or intermolecular hydrogen bonds) as well as from hydrophobic contributions. The formulation further comprises one or more pharmaceutically acceptable excipients and/or diluents inducing the formation of the matrix upon administration. The matrix provides for a slow absorption to the circulation from an injection site, and without being bound by theory, this slow absorption is due to the slow reversal of the matrix as protein concentration decreases at the injection site. The slow absorption profile provides for a flat PK profile, as well as convenient and comfortable administration regimen. For example, in various embodiments, the plasma concentration of the active agent over the course of days (*e.g.,* from 2 to about 60 days, or from about 4 to about 30 days) does not change by more than a factor of 10, or by more than a factor of about 5, or by more than a factor of about 3. Generally, this flat PK profile is seen over a plurality of (substantially evenly spaced) administrations, such as at least 2, at least about 5, or at least about 10 administrations of the formulation. In some embodiments, the slow absorption is exhibited by a Tmax (time to maximum plasma concentration) of greater than about 5 hours, greater than about 10 hours, greater than about 20 hours, greater than about 30 hours, or greater than about 50 hours.

The sustained release, or slow absorption from the injection site, is controlled by the amino acid sequence capable of forming a hydrogen-bonded matrix at the body temperature of the subject, as well as the components of the formulation.

In some embodiments, the amino acid sequence contains structural units that form hydrogen-bonds through protein backbone groups and/or side chain groups, and which may contribute hydrophobic interactions to matrix formation. In some embodiments, the amino acids side chains do not contain hydrogen bond donor groups, with hydrogen bonds being formed substantially through the protein backbone. Exemplary amino acids include proline, alanine, valine, glycine, and isoleucine, and similar amino acids. In some embodiments, the structural units are substantially repeating structural units, so as to create a substantially repeating structural motif, and substantially repeating hydrogen-bonding capability. In these and other embodiments, the amino acid sequence contains at least 10%, at least 20%, at least 40%, or at least 50% proline, which may be positioned in a substantially repeating pattern. In this context, a substantially repeating pattern means that at least 50% or at least 75% of the proline residues of the amino acid sequence are part of a definable structural unit. In still other embodiments, the amino acid sequence contains amino acids with hydrogen-bond donor side chains, such as serine, threonine, and/or tyrosine. In some embodiments, the repeating sequence may contain from one to about four proline residues, with remaining residues independently selected from non-polar residues, such as glycine, alanine, leucine, isoleucine, and valine. Non-polar or hydrophobic residues may contribute hydrophobic interactions to the formation of the matrix.

The amino acid sequences may form a "gel-like" state upon injection at a temperature higher than the storage temperature. Exemplary sequences have repeating peptide units, and/or may be relatively unstructured at the lower temperature, and achieve a hydrogen-bonded, structured, state at the higher temperature.

In some embodiments, the amino acid sequence capable of forming the matrix at body temperature is a peptide having repeating units of from four to ten amino acids. The repeating unit may form one, two, or three hydrogen bonds in the formation of the matrix. In certain embodiments, the amino acid sequence capable of forming the matrix at body temperature is an amino acid sequence of silk, elastin, collagen, or keratin, or mimic thereof, or an amino acid sequence disclosed in U.S. Patent 6,355,776, which is hereby incorporated by reference.

In certain embodiments, the amino acid sequence is an Elastin-Like-Protein (ELP) sequence. The ELP sequence comprises or consists of structural peptide units or sequences that are related to, or mimics of, the elastin protein. The ELP sequence is constructed from structural units of from three to about twenty amino acids, or in some embodiments, from four to ten amino acids, such as four, five or six amino acids. The length of the individual structural units may vary or may be uniform. Exemplary structural units include units defined by SEQ ID NOS: 1-12 (below), which may be employed as repeating structural units, including tandem-repeating units, or may be employed in some combination. Thus, the ELP may comprise or consist essentially of structural unit(s) selected from SEQ ID NOS: 1-12, as defined below.

In some embodiments, including embodiments in which the structural units are ELP units, the amino acid sequence comprises or consists essentially of from about 10 to about 500 structural units, or in certain embodiments about 50 to about 200 structural units, or in certain embodiments from about 80 to about 200 structural units, or from about 80 to about 150 structural units, such as one or a combination of units defined by SEQ ID NOS: 1-12. Thus, the structural units collectively may have a length of from about 50 to about 2000 amino acid residues, or from about 100 to about 800 amino acid residues, or from about 200 to about 700 amino acid residues, or from about 400 to about 600 amino acid residues.

The amino acid sequence may exhibit a visible and reversible inverse phase transition with the selected formulation. That is, the amino acid sequence may be structurally disordered and highly soluble in the formulation below a transition temperature (Tt), but exhibit a sharp (2-3°C range) disorder-to-order phase transition when the temperature of the formulation is raised above the Tt. In addition to temperature, length of the amino acid polymer, amino acid composition, ionic strength, pH, pressure, temperature, selected solvents, presence of organic solutes, and protein concentration may also affect the transition properties, and these may be tailored in the formulation for the desired absorption profile. Absorption profile can be easily tested by determining plasma concentration or activity of the active agent over time.

In certain embodiments, the ELP component(s) may be formed of structural units, including but not limited to:
(a) the tetrapeptide Val-Pro-Gly-Gly, or VPGG (SEQ ID NO: 1);
(b) the tetrapeptide Ile-Pro-Gly-Gly, or IPGG (SEQ ID NO: 2);
(c) the pentapeptide Val-Pro-Gly-X-Gly (SEQ ID NO: 3), or VPGXG, where X is any natural or non-natural amino acid residue, and where X optionally varies among polymeric or oligomeric repeats;
(d) the pentapeptide Ala-Val-Gly-Val-Pro, or AVGVP (SEQ ID NO: 4);
(e) the pentapeptide Ile-Pro-Gly-X-Gly, or IPGXG (SEQ ID NO: 5), where X is any natural or non-natural amino acid residue, and where X optionally varies among polymeric or oligomeric repeats;
(e) the pentapeptide Ile-Pro-Gly-Val-Gly, or IPGVG (SEQ ID NO: 6);
(f) the pentapeptide Leu-Pro-Gly-X-Gly, or LPGXG (SEQ ID NO: 7), where X is any natural or non-natural amino acid residue, and where X optionally varies among polymeric or oligomeric repeats;
(g) the pentapeptide Leu-Pro-Gly-Val-Gly, or LPGVG (SEQ ID NO: 8);
(h) the hexapeptide Val-Ala-Pro-Gly-Val-Gly, or VAPGVG (SEQ ID NO: 9);
(i) the octapeptide Gly-Val-Gly-Val-Pro-Gly-Val-Gly, or GVGVPGVG (SEQ ID NO: 10);
(j) the nonapeptide Val-Pro-Gly-Phe-Gly-Val-Gly-Ala-Gly, or VPGFGVGAG (SEQ ID NO: 11); and
(k) the nonapeptides Val-Pro-Gly-Val-Gly-Val-Pro-Gly-Gly, or VPGVGVPGG (SEQ ID NO: 12).

Such structural units defined by SEQ ID NOS:1-12 may form structural repeat units, or may be used in combination to form an ELP. In some embodiments, the ELP component is formed entirely (or almost entirely) of one or a combination of (e.g., 2, 3 or 4) structural units selected from SEQ ID NOS: 1-12. In other embodiments, at least 75%, or at least 80%, or at least 90% of the ELP component is formed from one or a combination of structural units selected from SEQ ID NOS: 1-12, and which may be present as repeating units.

In certain embodiments, the ELP contains repeat units, including tandem repeating units, of Val-Pro-Gly-X-Gly (SEQ ID NO: 3), where X is as defined above, and where the percentage of Val-Pro-Gly-X-Gly (SEQ ID NO: 3) units taken with respect to the entire ELP component (which may comprise structural units other than VPGXG (SEQ ID NO: 3)) is greater than about 50%, or greater than about 75%, or greater than about 85%, or greater than about 95% of the ELP. The ELP may contain motifs of 5 to 15 structural units (*e.g.* about 10 structural units) of SEQ ID NO: 3, with the guest residue X varying among at least 2 or at least 3 of the units in the motif. The guest residues may be independently selected, such as from non-polar or hydrophobic residues, such as the amino acids V, I, L, A, G, and W (and may be selected so as to retain a desired inverse phase transition property).

In some embodiments, the ELP may form a β-turn structure. Exemplary peptide sequences suitable for creating a β-turn structure are described in International Patent Application PCT/US96/05186, which is hereby incorporated by reference in its entirety. For example, the fourth residue (X) in the sequence VPGXG (SEQ ID NO: 3), can be altered without eliminating the formation of a β-turn.

The structure of exemplary ELPs may be described using the notation ELPk [XiYj-n], where k designates a particular ELP repeat unit, the bracketed capital letters are single letter amino acid codes and their corresponding subscripts designate the relative ratio of each guest residue X in the structural units (where applicable), and n describes the total length of the ELP in number of the structural repeats. For example, ELP1 [V5A2G3-10] designates an ELP component containing 10 repeating units of the pentapeptide VPGXG (SEQ ID NO: 3), where X is valine, alanine, and glycine at a relative ratio of about 5:2:3; ELP1 [K1V2F1-4] designates an ELP component containing 4 repeating units of the pentapeptide VPGXG (SEQ ID NO: 3), where X is lysine, valine, and phenylalanine at a relative ratio of about 1:2:1; ELP1 [K1V7F1-9] designates a polypeptide containing 9 repeating units of the pentapeptide VPGXG (SEQ ID NO: 3), where X is lysine, valine, and phenylalanine at a relative ratio of about 1:7:1; ELP1 [V-5] designates a polypeptide containing 5 repeating units of the pentapeptide VPGXG (SEQ ID NO:3), where X is valine; ELP1 [V-20] designates a polypeptide containing 20 repeating units of the pentapeptide VPGXG (SEQ ID NO: 3), where X is valine; ELP2 [5] designates a polypeptide containing 5 repeating units of the pentapeptide AVGVP (SEQ ID NO: 4); ELP3 [V-5] designates a polypeptide containing 5 repeating units of the pentapeptide IPGXG (SEQ ID NO: 5), where X is valine; ELP4 [V-5] designates a polypeptide containing 5 repeating units of the pentapeptide LPGXG (SEQ ID NO: 7), where X is valine.

With respect to ELP, the Tt is a function of the hydrophobicity of the guest residue. Thus, by varying the identity of the guest residue(s) and their mole fraction(s), ELPs can be synthesized that exhibit an inverse transition over a broad range. Thus, the Tt at a given ELP length may be decreased by incorporating a larger fraction of hydrophobic guest residues in the ELP sequence. Examples of suitable hydrophobic guest residues include valine, leucine, isoleucine, phenylalanine, tryptophan and methionine. Tyrosine, which is moderately hydrophobic, may also be used. Conversely, the Tt may be increased by incorporating residues, such as those selected from: glutamic acid, cysteine, lysine, aspartate, alanine, asparagine, serine, threonine, glycine, arginine, and glutamine.

For polypeptides having a molecular weight > 100,000, the hydrophobicity scale disclosed in PCT/US96/05186 (which is hereby incorporated by reference in its entirety) provides one means for predicting the approximate Tt of a specific ELP sequence. For polypeptides having a molecular weight <100,000, the Tt may be predicted or determined by the following quadratic function: Tt = M0 + M1X + M2X2 where X is the MW of the fusion protein, and M0 = 116.21; M1 = -1.7499; M2 = 0.010349.

The ELP in some embodiments is selected or designed to provide a Tt ranging from about 10 to about 37°C at formulation conditions, such as from about 20 to about 37°C, or from about 25 to about 37°C. In some embodiments, the transition temperature at physiological conditions (e*.g.,* 0.9% saline) is from about 34 to 36°C, to take into account a slightly lower peripheral temperature.

In certain embodiments, the amino acid sequence capable of forming the hydrogen-bonded matrix at body temperature comprises [VPGXG]₉₀, where each X is selected from V, G, and A, and wherein the ratio of V:G:A may be about 5:3:2. For example, the amino acid sequence capable of forming the hydrogen-bonded matrix at body temperature may comprise [VPGXG]₁₂₀, where each X is selected from V, G, and A, and wherein the ratio of V:G:A may be about 5:3:2. As shown herein, 120 structural units of this ELP can provide a transition temperature at about 37°C with about 5 to 15 mg/ml (*e.g.,* about 10 mg/ml) of protein. At concentrations of about 50 to about 100 mg/mL the phase transition temperature is about 35.5 degrees centigrade (just below body temperature), which allows for peripheral body temperature to be just less than 37°C.

Alternatively, the amino acid sequence capable of forming the matrix at body temperature comprises [VPGVG]₉₀, or [VPGVG]₁₂₀. As shown herein, 120 structural units of this ELP can provide a transition temperature at about 37°C with about 0.005 to about 0.05 mg/ml (*e.g.,* about 0.01 mg/ml) of protein.

Elastin-like-peptide (ELP) protein polymers and recombinant fusion proteins can be prepared as described in U.S. Patent Publication No. 2010/0022455, which is hereby incorporated by reference.

In other embodiments, the amino acid sequence capable of forming the matrix at body temperature may include a random coil or non-globular extended structure. For example, the amino acid sequence capable of forming the matrix at body temperature may comprise an amino acid sequence disclosed in U.S. Patent Publication No. 2008/0286808, WIPO Patent Publication No. 2008/155134, and U.S. Patent Publication No. 2011/0123487, each of which is hereby incorporated by reference.

For example, in some embodiments the amino acid sequence comprises an unstructured recombinant polymer of at least 40 amino acids. For example, the unstructured polymer may be defined where the sum of glycine (G), aspartate (D), alanine (A), serine (S), threonine (T), glutamate (E) and proline (P) residues contained in the unstructured polymer, constitutes more than about 80% of the total amino acids. In some embodiments, at least 50% of the amino acids are devoid of secondary structure as determined by the Chou-Fasman algorithm. The unstructured polymer may comprise more than about 100, 150, 200 or more contiguous amino acids. In some embodiments, the amino acid sequence forms a random coil domain. In particular, a polypeptide or amino acid polymer having or forming "random coil conformation" substantially lacks a defined secondary and tertiary structure.

In various embodiments, the intended subject is human, and the body temperature is about 37°C, and thus the therapeutic agent is designed to provide a sustained release at this temperature. A slow release into the circulation with reversal of hydrogen bonding and/or hydrophobic interactions is driven by a drop in concentration as the product diffuses at the injection site, even though body temperature remains constant. In other embodiments, the subject is a non-human mammal, and the therapeutic agent is designed to exhibit a sustained release at the body temperature of the mammal, which may be from about 30 to about 40°C in some embodiments, such as for certain domesticated pets (*e.g.,* dog or cat) or livestock (e.g., cow, horse, sheep, or pig). Generally, the Tt is higher than the storage conditions of the formulation (which may be from 10 to about 25°C, or from 15 to 22°C), such that the therapeutic agent remains in solution for injection.

The therapeutic agent is generally for "systemic delivery," meaning that the agent is not delivered locally to a pathological site or a site of action. Instead, the agent is absorbed into the bloodstream from the injection site, where the agent acts systemically or is transported to a site of action via the circulation.

In various embodiments, the active agent is a protein or peptide, which may have a short circulatory half-life, such as from about 30 seconds to about 1 hour. The therapeutic agent may be a recombinant fusion protein between the protein active agent and the amino acid sequence capable of forming the hydrogen-bonded matrix at the body temperature of the subject. Exemplary peptide active agents include GIP receptor agonists such as glucose-dependent insulinotropic peptide (GIP) or a derivative thereof. Further exemplary peptide active agents include GLP1 receptor agonists such as GLP-1 or derivative thereof (including GLP1 7-36 or GLP1 7-37), or exendin or derivative thereof. In other embodiments, the protein or peptide agent is a glucagon receptor agonist (including glucagon, oxyntomodulin or a derivative thereof). In some embodiments, the GLP-1 receptor agonist is a dual agonist having an amino acid sequence described in US 2011/0257092, which is hereby incorporated by reference in its entirety. Other dual or multi receptor agonists are described in US 2011/016602 and US 2010/00190701, each of which is hereby incorporated by reference, in particular with regard to the structures and sequences of GLP-1 receptor co-agonists described therein. Additional descriptions of GLP-1 receptor co-agonists can be found in Pocai A et al., Glucagon-Like Peptide 1/Glucagon Receptor Dual Agonism Reverses Obesity in Mice, Diabetes 58:2258-2266 (2009) and Patterson JT, et al., Functional association of the N-terminal residues with the central region in glucagon-related peptides, J. Pept. Sci. 17:659-666 (2011), each of which are hereby incorporated by reference in their entirety. In another embodiment, the invention provides for a co-formulation of any two of a GLP1 receptor agonist, a glucagon receptor agonist, and a GIP receptor agonist. In other embodiments, the protein or peptide agent is a VPAC2 selective agonist, such as vasoactive intestinal peptide (VIP) or a derivative thereof. Alternatively, the protein active agent is a clotting factor, such as Factor VII, Factor VIII, or Factor IX, or in other embodiments is insulin (*e.g.,* single chain insulin or an A chain or a B chain fusion protein, as described in U.S. Provisional Application No. 61/563,985, which is hereby incorporated by reference) or a monoclonal antibody or single chain antibody. Alternatively, the active agent is as described in U.S. Patent Publication No. 2011/0123487, which is hereby incorporated by reference. Exemplary therapeutic agents in accordance with the invention include GLP-1 (A8G,7-37) ELP1-120 (referred to herein as PB1023) or GLP-1 (A8G,7-37) ELP4-120 (PB1046). By providing a slow absorption from the injection site, renal clearance and degradation can be controlled, thereby achieving the desired pK profile.

In various embodiments, the invention encompasses doses and/or regimens such as those that do not induce substantial appetite suppression in a patient and/or those that do not induce substantial nausea in the patient, such as those described in PCT/US12/44383, which is hereby incorporated by reference.

In other embodiments, the therapeutic agent is a chemical conjugate between the active agent and the amino acid sequence capable of forming the matrix at the body temperature of the subject. For example, the active agent may be a chemotherapeutic agent, such as a chemotherapeutic agent selected from methotrexate, daunomycin, mitomycin, cisplatin, vincristine, epirubicin, fluorouracil, verapamil, cyclophosphamide, cytosine arabinoside, aminopterin, bleomycin, mitomycin C, democolcine, etoposide, mithramycin, chlorambucil, melphalan, daunorubicin, doxorubicin, tamoxifen, paclitaxel, vinblastine, camptothecin, actinomycin D, cytarabine, and combrestatin. Alternatively, the agent may be an immunogenic molecule, or an immunomodulator, or an anti-inflammatory agent, such as an agent described in U.S. Patent Publication No. 2009/0004104, which is hereby incorporated by reference in its entirety. Also, the agent may be an opioid molecule, such as, for example oxycodone, morphine, or codeine such as described in U.S. Provisional Application No. 61/597,898, which is hereby incorporated by reference. The chemical conjugate may be through a cleavable linker, for which numerous types are known in the art. *See* U.S. Patent No. 6,328,996, which is hereby incorporated by reference in its entirety.

The formulation comprises one or more pharmaceutically acceptable excipients and/or diluents inducing the formation of the matrix upon administration. For example, such excipients include salts, and other excipients that may act to stabilize hydrogen bonding. Exemplary salts include alkaline earth metal salts such as sodium, potassium, and calcium. Counter ions include chloride and phosphate. Exemplary salts include sodium chloride, potassium chloride, magnesium chloride, calcium chloride, and potassium phosphate.

The protein concentration in the formulation is tailored to drive, along with the excipients, the formation of the matrix at the temperature of administration. For example, higher protein concentrations help drive the formation of the matrix, and the protein concentration needed for this purpose varies depending on the ELP series used. For example, in embodiments using an ELP1-120, or amino acid sequences with comparable transition temperatures, the protein is present in the range of about 1 mg/mL to about 200 mg/mL, or is present in the range of about 5 mg/mL to about 125 mg/mL. The therapeutic agent may be present in the range of about 10 mg/mL to about 50 mg/mL, or about 15 mg/mL to about 30 mg/mL. In embodiments using an ELP4-120, or amino acid sequences with comparable transition temperatures, the protein is present in the range of about 0.005 mg/mL to about 10 mg/mL, or is present in the range of about 0.01 mg/mL to about 5 mg/mL.

The therapeutic agent is formulated at a pH, ionic strength, and generally with excipients sufficient to drive the formation of the matrix at body temperature (*e.g.,* 37°C, or at from 34 to 36°C in some embodiments). The therapeutic agent is generally prepared such that it does not form the matrix at storage conditions. Storage conditions are generally less than the transition temperature of the formulation, such as less than about 32°C, or less than about 30°C, or less than about 27°C, or less than about 25°C, or less than about 20°C, or less than about 15°C. For example, the formulation may be isotonic with blood or have an ionic strength that mimics physiological conditions. For example, the formulation may have an ionic strength of at least that of 25 mM Sodium Chloride, or at least that of 30 mM Sodium chloride, or at least that of 40 mM Sodium Chloride, or at least that of 50 mM Sodium Chloride, or at least that of 75 mM Sodium Chloride, or at least that of 100 mM Sodium Chloride, or at least that of 150 mM Sodium Chloride. In certain embodiments, the formulation has an ionic strength less than that of 0.9% saline. In some embodiments, the formulation comprises two or more of calcium chloride, magnesium chloride, potassium chloride, potassium phosphate monobasic, sodium chloride, and sodium phosphate dibasic. The liquid formulation may comprise the components listed in **Table 4, Table 5,** or **Table 6,** and can be stored refrigerated or at room temperature.

The formulation can be packaged in the form of pre-dosed pens or syringes for administration once per week, twice per week, or from one to eight times per month, or alternatively filled in conventional vial and the like.

In exemplary embodiments, the invention provides a sustained release pharmaceutical formulation that comprises a therapeutic agent, the therapeutic agent (*e.g.,* a peptide or protein therapeutic agent) comprising an active agent and an amino acid sequence comprising [VPGXG]₉₀, or [VPGXG]₁₂₀, where each X is selected from V, G, and A. V, G, and A may be present at a ratio of about 5:3:2. Alternatively, the amino acid sequence comprises [VPGVG]90 or [VPGVG]120. The formulation further comprises one or more pharmaceutically acceptable excipients and/or diluents for formation of a reversible matrix from an aqueous form upon administration to a human subject. The active agent in certain embodiments is GLP-1 or derivative thereof (*e.g.,* GLP-1, A8G, 7-37), or vasoactive intestinal peptide (VIP) or a derivative thereof (*e.g.,* having an N-terminal moiety such as a Methionine), or oxyntomodulin of a derivative thereof, or insulin or a derivative thereof. GLP-1 and derivatives thereof are disclosed in U.S. Patent Publication No. 2011/0123487, which is hereby incorporated by reference. VIP and derivatives thereof are disclosed in U.S. Patent Publication No. 2011/0178017, which is hereby incorporated by reference. Insulin and derivatives thereof are described in U.S. Provisional Application No. 61/563,985, which is hereby incorporated by reference

In these embodiments, the therapeutic agent may be present in the range of about 0.5 mg/mL to about 200 mg/mL, or is present in the range of about 5 mg/mL to about 125 mg/mL. The therapeutic agent is present in the range of about 10 mg/mL to about 50 mg/mL, or the range of about 15 mg/mL to about 30 mg/mL The formulation may have an ionic strength of at least that of 25 mM Sodium Chloride, or at least that of 30 mM sodium Chloride, or at least that of 40 mM Sodium Chloride, or at that least that of 50 mM Sodium Chloride, or at least that of 75 mM Sodium Chloride, or at least that of 100 mM Sodium Chloride. The formulation may have an ionic strength less than that of about 0.9% saline. The formulation comprises two or more of calcium chloride, magnesium chloride, potassium chloride, potassium phosphate monobasic, sodium chloride, and sodium phosphate dibasic. The formulation may comprise the components listed in **Table 4, Table 5,** or **Table 6.**

Other formulation components for achieving the desired stability, for example, may also be employed. Such components include one or more amino acids or sugar alcohol (*e.g.,* mannitol), preservatives, and buffering agents, and such ingredients are well known in the art.

In another aspect, the invention provides a method for delivering a sustained release regimen of an active agent. The method comprises administering the formulation described herein to a subject in need, wherein the formulation is administered from about 1 to about 8 times per month. For example, the active agent may be GLP-1 or an analog thereof, and is administered in a method described in U.S. Patent Application No. 13/534,836, which is hereby incorporated by reference. For example, the therapeutic agent may be GLP-1 7-36 or 7-37, alternatively having Gly at position 8, fused to ELP1 (*e*.*g*., having from about 90 to about 150 ELP units). The GLP-1 fusion may be used for the treatment of diabetes (type 1 or 2), metabolic disease, or obesity, for example, by administering to a patient in need. Alternatively, the active agent is VIP or an analog thereof, and is administered in a method described in U.S. Patent Publication No. 2011/0178017, which is hereby incorporated by reference. The VIP may have an additional moiety such as Methionine at the N-terminus to alter the receptor binding profile, as also described in U.S. Patent Publication No. 2011/0178017, which description is hereby incorporated by reference. The VIP may be fused to ELP1 (having from about 90 to about 150 ELP units). The VIP active agent finds use in a method of treating a condition selected from uncontrolled or resistant hypertension, or pulmonary arterial hypertension (PAH), and chronic obstructive pulmonary disease (COPD), among others.

In some embodiments, the formulation is administered about weekly, and may be administered subcutaneously or intramuscularly. In some embodiments, the site of administration is not a pathological site, for example, is not the intended site of action.

In various embodiments, the plasma concentration of the active agent does not change by more than a factor of 10, or a factor of about 5, or a factor of about 3 over the course of a plurality of administrations, such as at least 2, at least about 5, or at least about 10 administrations of the formulation. The administrations are substantially evenly spaced, such as, for example, about daily, or about once per week, or from one to about five times per month.

In certain embodiments, the subject is a human, but in other embodiments may be a non-human mammal, such as a domesticated pet (*e.g.,* dog or cat), or livestock or farm animal (*e.g.,* horse, cow, sheep, or pig).

### EXAMPLES

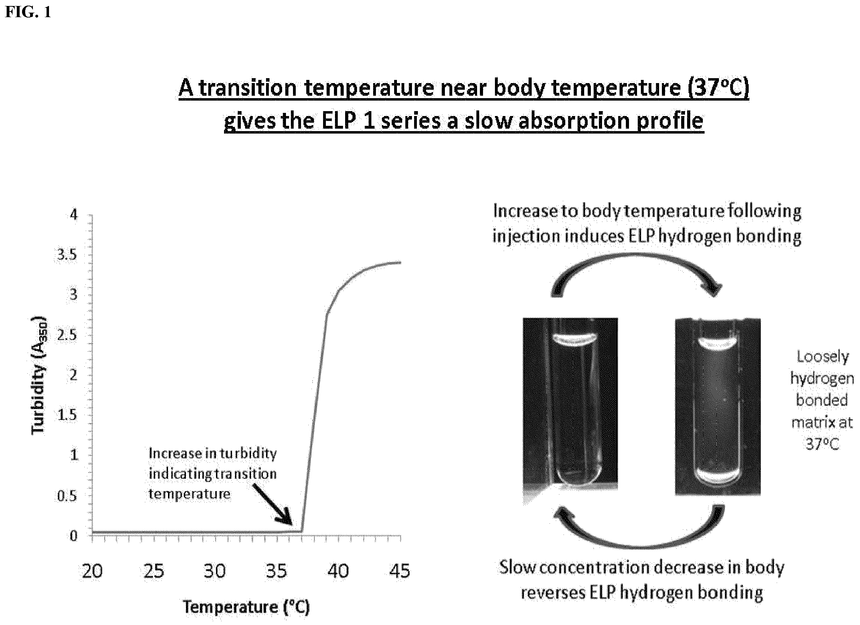

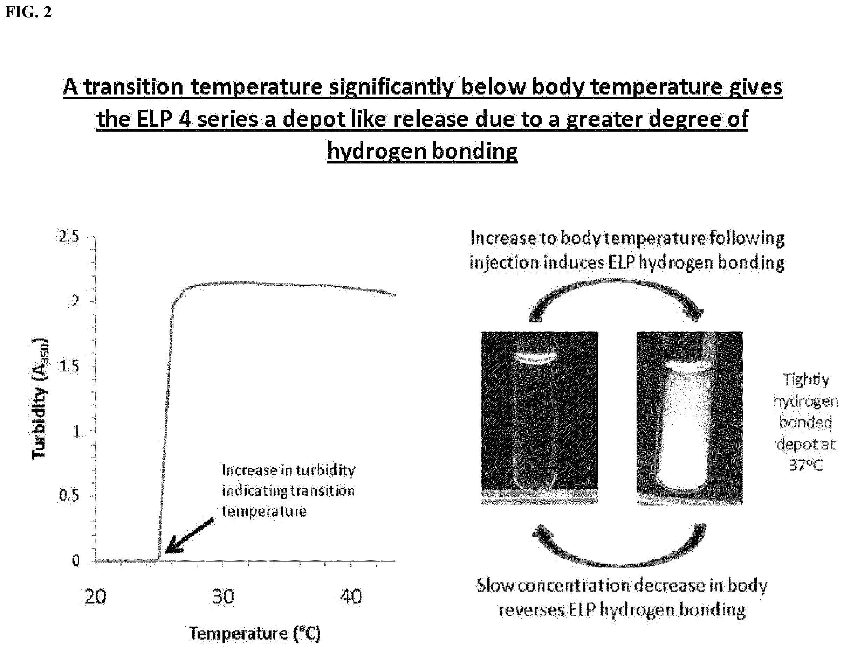

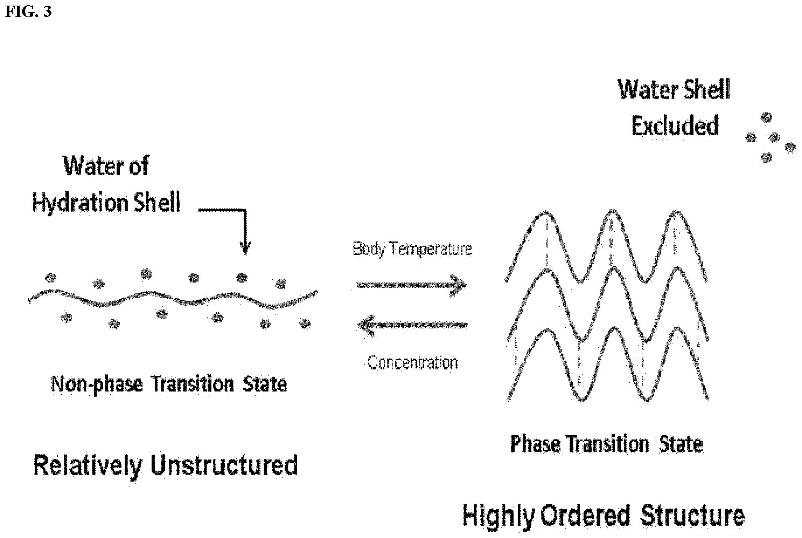

The phase transition property exhibited by certain amino acid sequences is illustrated in **Figure 1** (for ELP1) and **Figure 2** (for ELP4). Phase transition can be observed as an increase in turbidity. **Figure 3** illustrates, without wishing to be bound by theory, a potential mechanism for phase transition, driven by exclusion of a water shell and formation of hydrogen bonds at a temperature above the phase transition temperature for a given concentration.

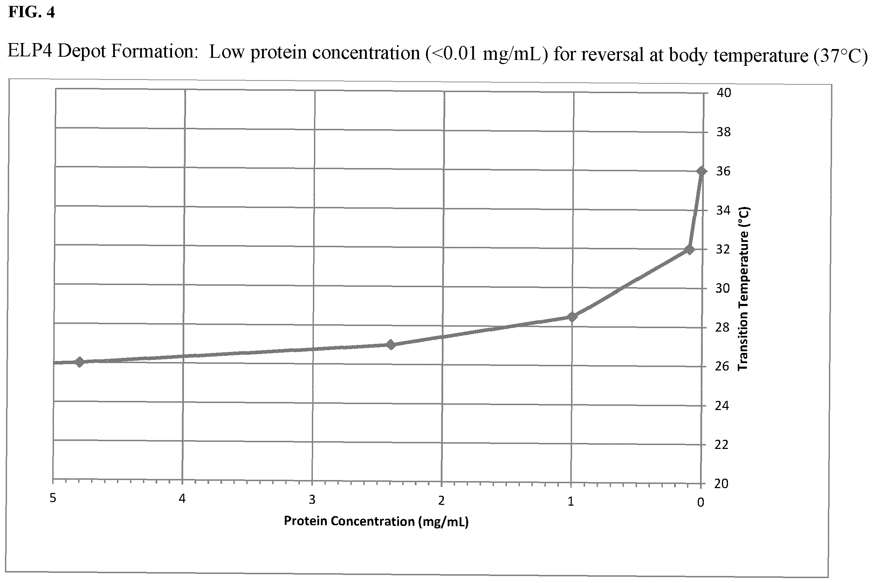

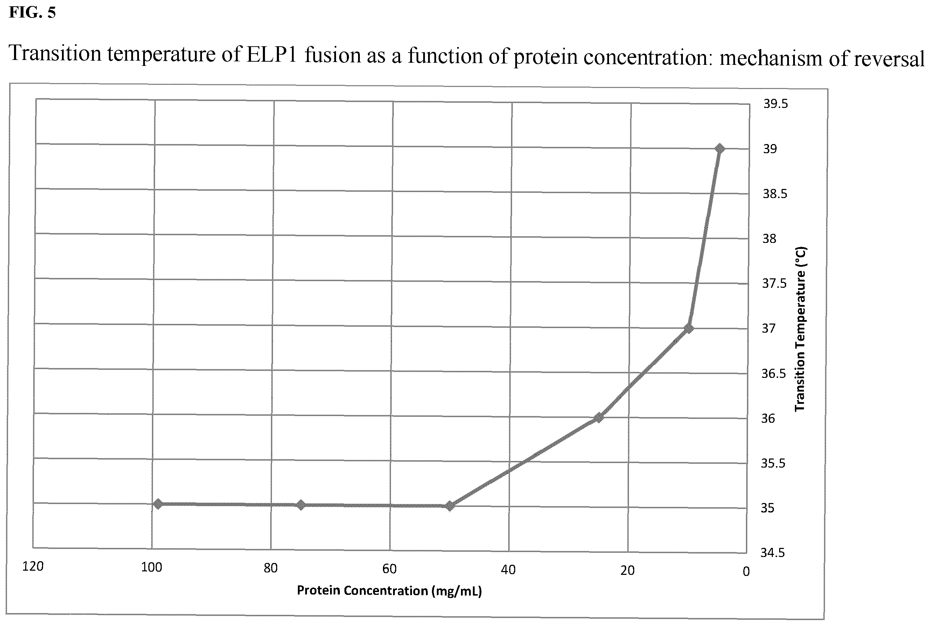

**Figure 4** shows that the ELP4 series (about 120 structural units) transitions at 37°C at a protein concentration of less than about 0.01 mg/mL, allowing for sustained release formulations of low protein concentration. At higher concentrations the sustained release will be sufficiently slow to provide a depot like formulation. **Figure 5** shows that the ELP1 series transitions between 35 and 37°C at relatively high protein concentration of about 10 mg/mL to about 100 mg/mL, or more, allowing for sustained release formulations with relatively high amounts of active agents.

Various formulations were prepared for PB1023 (GLP-1, A8G,7-37, ELP1-120) and PB1046 (GLP-1, A8G,7-37, ELP4-120), at varying protein concentrations and ionic strength. Transition induced by 37°C water bath was tested.

**Table 1** shows determination of phase transition for formulations of PB1023 and PB1046, varied by protein concentration and ionic strength. As shown, formulations of at least 50 mg/mL PB1023 and with an ionic strength of at least that of 10 mM His and 55 mM NaCl, transitioned at 37°C (with an approximate transition temperature of 35.5°C). A formulation of 25 mg/mL of PB1023 and an ionic strength of about normal saline also transitioned at 37°C. Formulations even as low as 1 mg/mL of PB1046 and having an ionic strength similar to normal saline transitioned at 37°C.

As shown in **Table 2,** Formulations of 25 mg/ml PB1023 in either: normal saline, DPBS, or 1x phosphate buffered saline, were sufficient to generate the desired transition property. Water alone did not support the desired transition property.

As shown in **Table 3,** a formulation of 25 mg/ml PB1023 transitions at 37°C with an ionic strength equal to 50 mM NaCl.

**Table 4, Table 5,** and **Table 6** show some buffer formulations in accordance with certain embodiments of the invention.

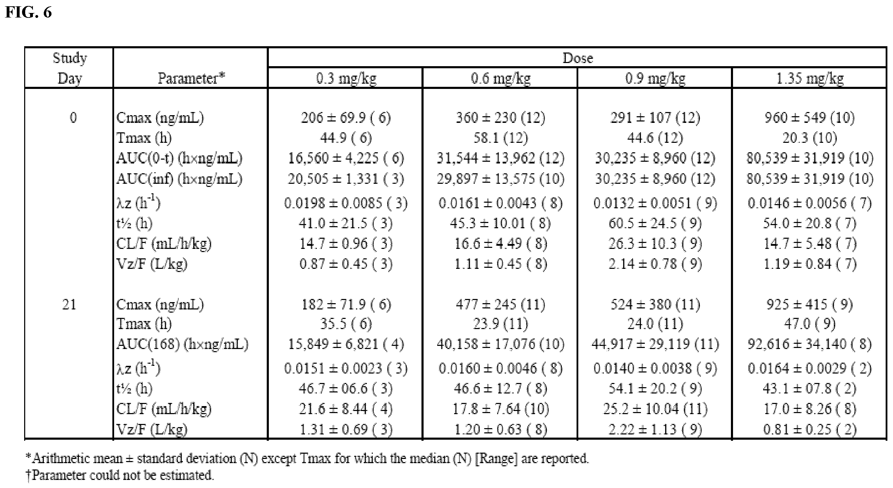

**Figure 6** shows a summary of pharmacokinetic parameters for GLP-1/ELP1-120 (also referred to herein as PB1023 or Glymera) after SC administration of 0.3, 0.6, 0.9 and 1.35 mg/kg to adult subjects with type 2 diabetes mellitus.

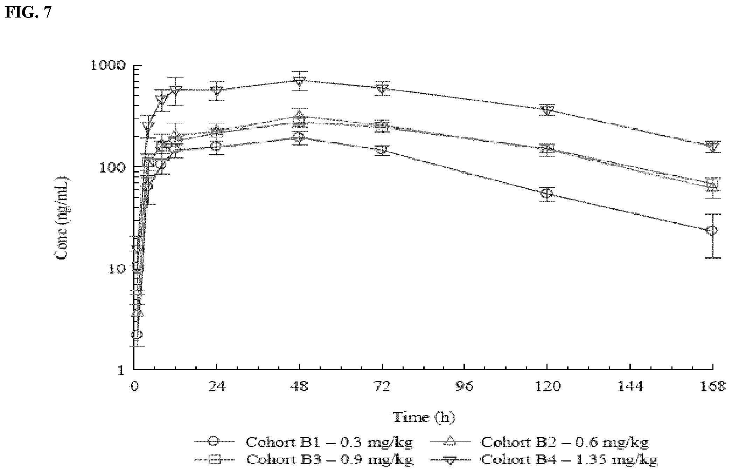

**Figure 7** shows the mean serum concentrations of GLP-1/ELP1-120 (also referred to herein as PB1023 or Glymera) after s.c. administration on day 0 of 0.3, 0.6, 0.9 and 1.35 mg/kg to adult subjects with type 2 diabetes mellitus (semi-logarithmic axes).

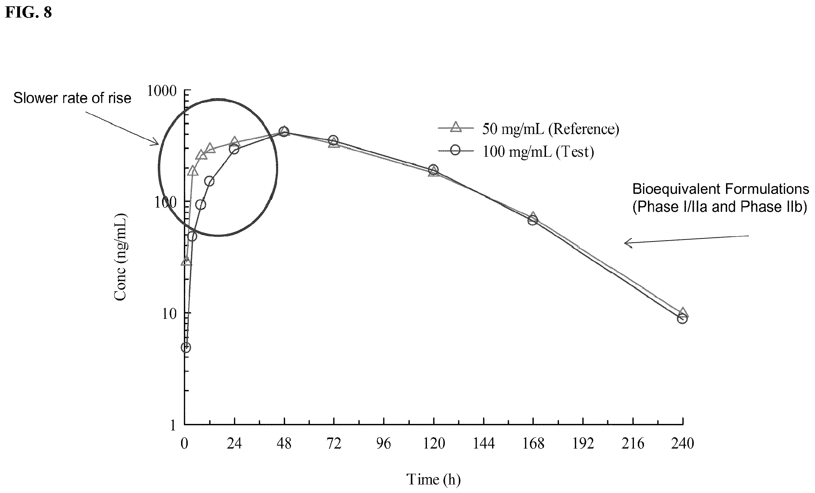

**Figure 8** shows a type 2 diabetes mellitus: Glymera program overview pharmacokinetics crossover study. Mean serum concentrations of Glymera following s.c. administration of 90 mg as 50 mg/mL and 100 mg/mL formulations to adult subjects with type 2diabetes mellitus are shown (semi-logarithmic axes). It is noted that that the time courses for mean serum distribution for the 50 mg/mL and 100 mg/mL are nearly equivalent on the whole, except that the 100 mg/mL dose bursts into the blood stream slower than the 50 mg/mL dose (*i.e.* the 100 mg/mL data set has a slower rate of rise).

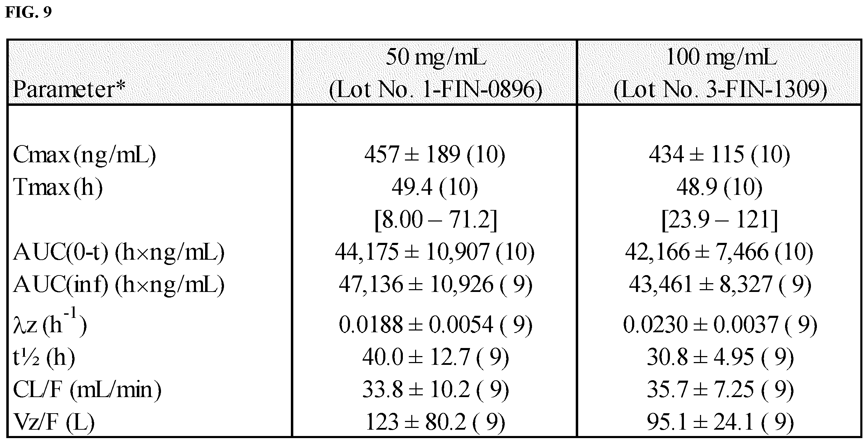

**Figure 9** shows a summary of pharmacokinetic parameters for ELP1-120 (also referred to herein as PB1023 or Glymera) after s.c. administration of 90 mg as 50 mg/mL and 100 mg/mL formulations to adult subjects with type 2 diabetes mellitus.

**Table 1: Initial Transition Experiments Using a 37°C Waterbath and Visual Interpretation of Results**

| **Drug/Formulation** | **Dilution Buffer** | **Final Concentration/ Formulation** | **Transition in 37°C waterbath** | **Cary Transition Temperature** |
|---|---|---|---|---|
| 100mg/mL PB1023 | NA | 100mg/mL PB1023 | Yes | ∼34.9°C |
| 20mM His, 110mM NaCl | | 20mM His, 110mM NaCl | | |
| 100mg/mL PB1023 | Water | 90mg/mL | Yes | |
| 20mM His, 110mM NaCl | | 18mM His, 99mM NaCl | | |
| 100mg/mL PB1023 | Water | 80mg/mL | Yes | |
| 20mM His, 110mM NaCl | | 16mM His, 88mM NaCl | | |
| 100mg/mL PB1023 | Water | 50mg/mL | Yes | |
| 20mM His, 110mM NaCl | | 10mM His, 55mMNaCl | | |
| 50mg/mL PB1023 | NA | 50mg/mL PB1023 | No | ∼49°C |
| 20mM Histidine | | 20mM Histidine | | |
| 50mg/mL PB1023 | Normal Saline (0.9% NaCl) | 25mg/mL | Yes | |
| 20mM Histidine | | 10mM Histidine, 75mM NaCl | | |
| 40mg/mL PB1046 | NA | 40mg/mL PB1046 | Yes | |
| 20mM His, 75mM NaCl | | 20mM His, 75mM NaCl | | |
| 40mg/mL PB1046 | Normal Saline (0.9% NaCl) | 12mg/mL PB1046 | Yes | |
| 20mM His, 75mM NaCl | | | | |
| 40mg/mL PB1046 | Normal Saline (0.9% NaCl) | 1mg/mL PB1046 | Yes | |
| 20mM His, 75mM NaCl | | | | |

**Table 2: Transition Temperature Experiments Using Various Dilution Buffers**

| **Drug/Formulation** | **Dilution Buffer** | **Final Concentration** | **Transition in 37°C waterbath** | **Cary Transition Temperature** |
|---|---|---|---|---|
| 50mg/mL PB1023 | Water | 25mg/mL PB1023 | No | ∼51.1 |
| 20mM Histidine | | | | |
| 50mg/mL PB1023 | Normal Saline (0.9% NaCl) | 25mg/mL PB1023 | Yes | ∼36.5 |
| 20mM Histidine | | | | |
| 50mg/mL PB1023 | DPBS w/ Mg and Ca | 25mg/mL PB1023 | Yes | |
| 20mM Histidine | | | | |
| 50mg/mL PB1023 | DPBS w/out Mg and Ca | 25mg/mL PB1023 | Yes | |
| 20mM Histidine | | | | |
| 50mg/mL PB1023 | 1X PBS | 25mg/mL PB1023 | Yes | |
| 20mM Histidine | | | | |

**Table 3: Transition Experiments Varying Salt Concentration**

| **Drug/Formulation** | **Dilution Buffer** | **Final Concentration/ Formulation** | **Transition in 37°C waterbath** | **Cary Transition Temperature** |
|---|---|---|---|---|
| 50mg/mL PB1023 | NaCl and Water | 25mg/mL PB1023 | Yes | ∼37°C |
| 20mM Histidine | | 50mM NaCl | | |
| 50mg/mL PB1023 | NaCl and Water | 25mg/mL PB1023 | | ∼37°C |
| 20mM Histidine | | 40mM NaCl | | |
| 50mg/mL PB1023 | NaCl and Water | 25mg/mL PB1023 | | ∼37°C |
| 20mM Histidine | | 30mM NaCl | | |
| 50mg/mL PB1023 | NaCl and Water | 25mg/mL PB1023 | Not Visible | ∼37°C |
| 20mM Histidine | | 25mM NaCl | | |
| 50mg/mL PB1023 | NaCl and Water | 25mg/mL PB1023 | Not Visible | |
| 20mM Histidine | | 12.5mM NaCl | | |
| 50mg/mL PB1023 | NaCl and Water | 25mg/mL PB1023 | | ∼37°C |
| 20mM Histidine | | 10mM NaCl | | |
| 50mg/mL PB1023 | NaCl and Water | 25mg/mL PB1023 | Not Visible | |
| 20mM Histidine | | 6.25mM NaCl | | |
| 50mg/mL PB1023 | NaCl and Water | 25mg/mL PB1023 | Not Visible | |
| 20mM Histidine | | 3.125mM NaCl | | |
| 50mg/mL PB1023 | NaCl and Water | 25mg/mL PB1023 | Not Visible | |
| 20mM Histidine | | 1.56mM NaCl | | |
| 50mg/mL PB1023 | NaCl and Water | 25mg/mL PB1023 | | ∼40.3C |
| 20mM Histidine | | 1mM NaCl | | |
| 50mg/mL PB1023 | NaCl and Water | 25mg/mL PB1023 | Not Visible | |
| 20mM Histidine | | 0.78mM NaCl | | |

**Table 4: Buffer Formulation-DPBS with Mg and Ca**

| **COMPONENTS** | **Molecular Weight** | **Concentration (mg/L)** | **mM** |
|---|---|---|---|
| Inorganic Salts | | | |
| Calcium Chloride (CaCl₂) (anhyd.) | 111 | 100 | 0.901 |
| Magnesium Chloride (MgCl₂-6H2O) | 203 | 100 | 0.493 |
| Potassium Chloride (KCl) | 75 | 200 | 2.67 |
| Potassium Phosphate monobasic (KH₂PO₄) | 136 | 200 | 1.47 |
| Sodium Chloride (NaCl) | 58 | 8000 | 137.93 |
| Sodium Phosphate dibasic (Na₂HPO₄-7H₂O) | 268 | 2160 | 8.06 |

**Table 5: Buffer Formulation-DPBS without Mg and Ca**

| **COMPONENTS** | **Molecular Weight** | | **Concentration (mg/L)** | **mM** |
|---|---|---|---|---|
| Inorganic Salts | | | | |
| Potassium Chloride (KCl) | | 75 | 200 | 2.67 |
| Potassium Phosphate monobasic (KH₂PO₄) | | 136 | 200 | 1.47 |
| Sodium Chloride (NaCl) | | 58 | 8000 | 137.93 |
| Sodium Phosphate dibasic (Na₂HPO₄-7H₂O) | | 268 | 2160 | 8.06 |

**Table 6: Buffer Formulation-1x PBS pH 7.4**

| **COMPONENTS** | **Molecular Weight** | **Concentration (mg/L)** | **mM** |
|---|---|---|---|
| Inorganic Salts | | | |
| Potassium Phosphate monobasic (KH₂PO₄) | 136 | 144 | 1.06 |
| Sodium Chloride (NaCl) | 58 | 9000 | 155.17 |
| Sodium Phosphate dibasic (Na₂HPO₄-7H₂O) | 268 | 795 | 2.97 |

### CLAUSES

1. A sustained release pharmaceutical formulation comprising:
   a therapeutic agent for systemic administration, the therapeutic agent comprising an active agent and an amino acid sequence capable of forming a reversible matrix at the body temperature of a subject, the reversible matrix formed of hydrogen bonds and/or hydrophobic interactions, and
   one or more pharmaceutically acceptable excipients and/or diluents inducing the formation of the matrix upon administration.
2. The pharmaceutical formulation of clause 1, wherein the formulation provides slow absorption from an injection site upon administration.
3. The pharmaceutical formulation of clause 2, wherein the formulation provides a flat PK profile upon administration, as compared to the PK profile for the active agent in the absence of the amino acid sequence forming a reversible matrix.
4. The pharmaceutical formulation of clause 3, wherein the PK profile has a shallow Cmax and/or low ratio of peak to trough and/or long Tmax.
5. The pharmaceutical formulation of any one of clauses 1 to 4, wherein the formation of the matrix reverses as protein concentration decreases.
6. The pharmaceutical formulation of any one of clauses 1 to 5, wherein the amino acid sequence capable of forming the matrix at or around body temperature is a repeating peptide sequence having repeating units of from four to ten amino acids.
7. The pharmaceutical formulation of clause 6, wherein the repeating unit forms one, two, or three hydrogen bonds in the formation of the matrix.
8. The pharmaceutical formulation of clause 6, wherein the amino acid sequence capable of forming the matrix at body temperature is an amino acid sequence of silk, elastin, collagen, or keratin, or mimic thereof.
9. The pharmaceutical formulation of any one of clauses 1 to 6, wherein the amino acid sequence capable of forming the matrix at body temperature comprises [VPGXG]₉₀, where each X is selected from V, G, and A, and wherein the ratio of V:G:A may be about 5:3:2.
10. The pharmaceutical formulation of clause 9, wherein the amino acid sequence capable of forming the matrix at body temperature comprises [VPGXG]₁₂₀, where each X is selected from V, G, and A, and wherein the ratio of V:G:A may be about 5:3:2.
11. The pharmaceutical formulation of any one of clauses 1 to 6, wherein the amino acid sequence capable of forming the matrix at body temperature comprises [VPGVG]₉₀.
12. The pharmaceutical formulation of any one of clauses 1 to 6, wherein the amino acid sequence capable of forming the matrix at body temperature is an elastin-like-peptide (ELP) sequence.
13. The pharmaceutical formulation of any one of clauses 1 to 5, wherein the amino acid sequence capable of forming the matrix at body temperature forms a random coil or non-globular extended structure.
14. The pharmaceutical formulation of any one of clauses 1 to 5, wherein the amino acid sequence capable of forming the matrix at body temperature is includes a random coil, or a non-globular extended structure.
15. The pharmaceutical formulation of any one of clauses 1 to 14, wherein the subject is human, and the body temperature is about 37°C.
16. The pharmaceutical formulation of any one of clauses 1 to 14, wherein the subject is a non-human mammal.
17. The pharmaceutical formulation of any one of clauses 1 to 16, wherein the active agent is a protein.
18. The pharmaceutical formulation of clause 17, wherein the therapeutic agent is a recombinant fusion protein between the protein active agent and the amino acid sequence capable of forming the matrix at the body temperature of the subject.
19. The pharmaceutical formulation of clause 18, wherein the protein active agent has a circulatory half-life in the range of from about 30 seconds to about 10 hour, or about 30 seconds to about 1 hour.
20. The pharmaceutical formulation of clause 19, wherein the active agent is a GLP-1 receptor agonist or derivative thereof, a VPAC2 selective agonist or a derivative thereof, a GIP receptor agonist or a derivative thereof or a glucagon receptor agonist or a derivative thereof.
21. The pharmaceutical formulation of clause 19, wherein the formulation is a co-formulation comprising at least two of a GLP1 receptor agonist, a glucagon receptor agonist, and a GIP receptor agonist
22. The pharmaceutical formulation of clause 19, wherein the protein active agent is a clotting factor, where the clotting factor may be Factor VII, Factor VIII, or Factor IX.
23. The pharmaceutical formulation of any one of clauses 1 to 22, wherein the active agent is selected from GLP-1 (A8G,7-37) ELP1-120 and GLP-1 (A8G,7-37) ELP4-120 (PB1046).
24. The pharmaceutical formulation of clause 19, wherein the protein active agent is insulin or a derivative thereof.
25. The pharmaceutical formulation of any one of clauses 1 to 17, wherein the therapeutic agent is a chemical conjugate between the active agent and the amino acid sequence capable of forming the matrix at the body temperature of the subject.
26. The pharmaceutical formulation of clause 25, wherein the active agent is a chemotherapeutic agent, such as a chemotherapeutic agent selected from methotrexate, daunomycin, mitomycin, cisplatin, vincristine, epirubicin, fluorouracil, verapamil, cyclophosphamide, cytosine arabinoside, aminopterin, bleomycin, mitomycin C, democolcine, etoposide, mithramycin, chlorambucil, melphalan, daunorubicin, doxorubicin, tamoxifen, paclitaxel, vinblastine, camptothecin, actinomycin D, cytarabine, and combrestatin.
27. The pharmaceutical formulation of clause 25, wherein the agent is an immunogenic molecule, or is an immunomodulator, or is an anti-inflammatory agent.
28. The pharmaceutical formulation of any one of clauses 1 to 27, wherein the therapeutic agent is present in the range of about 0.5 mg/mL to about 200 mg/mL.
29. The pharmaceutical formulation of clause 28, wherein the therapeutic agent is present in the range of about 5 mg/mL to about 125 mg/mL.
30. The pharmaceutical formulation of clause 28, wherein the therapeutic agent is present in the range of about 10 mg/mL to about 50 mg/mL, or about 15 mg/mL to about 30 mg/mL.
31. The pharmaceutical composition of any one of clauses 1 to 30, wherein the therapeutic agent does not form the phase-transitioned matrix at storage conditions.
32. The pharmaceutical composition of clause 31, wherein the storage conditions are less than about 40°C, or less than about 37°C, or less than about 30°C, or less than about 27°C, or less than about 25°C.
33. The pharmaceutical formulation of any one of clauses 1 to 32, wherein the formulation has an ionic strength of at least that of 25 mM Sodium Chloride, or at least that of 30 mM Sodium chloride, or at least that of 40 mM Sodium Chloride, or at least that of, 50 mM Sodium Chloride, or at least that of 75 mM Sodium Chloride, or at least that of 100 mM Sodium Chloride, or at least that of 150 mM Sodium Chloride.
34. The pharmaceutical formulation of any one of clauses 1 to 33, wherein the formulation has an ionic strength of about 0.9% saline or less.
35. The pharmaceutical formulation of clause 34, wherein the formulation comprises two or more of calcium chloride, magnesium chloride, potassium chloride, potassium phosphate monobasic, sodium chloride, and sodium phosphate dibasic.
36. The pharmaceutical formulation of clause 35, wherein the formulation comprises the components listed in Table 4, Table 5, or Table 6.
37. The pharmaceutical formulation of any one of clauses 1 to 36, wherein the formulation is packaged in the form of pre-dosed pens or syringes for administration once per week, twice per week, or from one to five times per month.
38. A sustained release pharmaceutical formulation comprising:
   a therapeutic agent, the therapeutic agent comprising an active agent and an amino acid sequence comprising [VPGXG]₉₀, where each X is selected from V, G, and A, and
   one or more pharmaceutically acceptable excipients and/or diluents for formation of a reversible matrix from an aqueous form upon administration to a human subject.
39. The pharmaceutical formulation of clause 38, wherein the formulation provides slow absorption from an injection site upon administration.
40. The pharmaceutical formulation of clause 39, wherein the formulation provides a flat PK profile upon administration, as compared to the PK profile for the active agent in the absence of said amino acid sequence.
41. The pharmaceutical formulation of clause 40, wherein the PK profile has a shallow Cmax and/or low ratio of peak to trough and/or a long Tmax.
42. The pharmaceutical formulation of any one of clauses 37 to 41, wherein the formation of the matrix reverses as protein concentration decreases.
43. The pharmaceutical formulation of clause 42, wherein the amino acid sequence comprises [VPGXG]₁₂₀, where each X is selected from V, G, and A.
44. The pharmaceutical formulation of any one of clauses 37 to 43, wherein V, G, and A are at a ratio of about 5:3:2.
45. The pharmaceutical formulation of any one of clauses 37 to 44, wherein the active agent is a protein.
46. The pharmaceutical formulation of clause 45, wherein the therapeutic agent is a recombinant fusion protein between the protein active agent and said amino acid sequence.
47. The pharmaceutical formulation of clause 46, wherein the protein active agent has a circulatory half-life in the range of from about 30 seconds to about 10 hours, or about 30 second to about 1 hour.
48. The pharmaceutical formulation of clause 46, wherein the active agent is a GLP-1 receptor agonist or derivative thereof, a VPAC2 selective agonist or a derivative thereof, a GIP receptor agonist or a derivative thereof or a glucagon receptor agonist or a derivative thereof.
49. The pharmaceutical formulation of clause 46, wherein the formulation is a co-formulation comprising at least two of a GLP1 receptor agonist, a glucagon receptor agonist, and a GIP receptor agonist
50. The pharmaceutical formulation of clause 46, wherein the protein active agent is a clotting factor, where the clotting factor may be Factor VII, Factor VIII, or Factor IX.
51. The pharmaceutical formulation of clause 46, wherein the protein active agent is insulin or a derivative thereof.
52. The pharmaceutical formulation of any one of clauses 38 to 51, wherein the active agent is selected from GLP-1 (A8G, 7-37) ELP1-120 and GLP-1 (A8G,7-37) ELP4-120 (PB1046).
53. The pharmaceutical formulation of any one of clauses 38 to 52, wherein the therapeutic agent is a chemical conjugate between the active agent and said amino acid sequence.
54. The pharmaceutical formulation of clause 53, wherein the active agent is a chemotherapeutic agent, such as a chemotherapeutic agent selected from methotrexate, daunomycin, mitomycin, cisplatin, vincristine, epirubicin, fluorouracil, verapamil, cyclophosphamide, cytosine arabinoside, aminopterin, bleomycin, mitomycin C, democolcine, etoposide, mithramycin, chlorambucil, melphalan, daunorubicin, doxorubicin, tamoxifen, paclitaxel, vinblastine, camptothecin, actinomycin D, cytarabine, and combrestatin.
55. The pharmaceutical formulation of clause 53, wherein the agent is an immunogenic molecule, or is an immunomodulator, or is an anti-inflammatory agent.
56. The pharmaceutical formulation of any one of clauses 38 to 56, wherein the therapeutic agent is present in the range of about 0.5 mg/mL to about 200 mg/mL.
57. The pharmaceutical formulation of clause 56, wherein the therapeutic agent is present in the range of about 5 mg/mL to about 125 mg/mL.
58. The pharmaceutical formulation of clause 56, wherein the therapeutic agent is present in the range of about 10 mg/mL to about 50 mg/mL, or the range of about 15 mg/mL to about 30 mg/mL.
59. The pharmaceutical composition of any one of clauses 38 to 58, wherein the therapeutic agent does not form the matrix at storage conditions.
60. The pharmaceutical composition of clause 59, wherein the storage conditions are less than about 40°C, or less than about 37°C, or less than about 30°C, or less than about 27°C, or less than about 25°C.
61. The pharmaceutical formulation of any one of clauses 38 to 60, wherein the formulation has an ionic strength of at least that of 25 mM Sodium Chloride, or at least that of 30 mM sodium Chloride, or at least that of 40 mM Sodium Chloride, or at that least that of 50 mM Sodium Chloride, or at least that of 75 mM Sodium Chloride, or at least that of 100 mM Sodium Chloride.
62. The pharmaceutical formulation of any one of clauses 61, wherein the formulation has an ionic strength of about 0.9% saline or less.
63. The pharmaceutical formulation of clause 61, wherein the formulation comprises two or more of calcium chloride, magnesium chloride, potassium chloride, potassium phosphate monobasic, sodium chloride, and sodium phosphate dibasic.
64. The pharmaceutical formulation of clause 63, wherein the formulation comprises the components listed in Table 4, Table 5, or Table 6.
65. The pharmaceutical formulation of any one of clauses 38 to 64, wherein the formulation is packaged in the form of pre-dosed pens or syringes for administration once per week, twice per week, or from one to five times per month.
66. A method for delivering a sustained release regimen of an active agent, comprising, administering the formulation of any one of clauses 1 to 65 to a subject in need, wherein the formulation is administered from about 1 to about 8 times per month.
67. The method of clause 66, wherein the active agent is GLP-1 or an analog thereof, and is administered about 1 to about 8 times per month.
68. The method of clause 66, wherein the active agent is VIP or an analog thereof, and is administered about 1 to about 8 times per month.
69. The method of any one of clauses 66 to 68, wherein the formulation is administered about weekly.
70. The method of any one of clauses 66 to 69 wherein the formulation is administered subcutaneously or intramuscularly.
71. The method of any one of clauses 66 to 69, wherein the site of administration is not a pathological site.

## Claims

1. A sustained release pharmaceutical formulation comprising:
a therapeutic agent for systemic administration comprising an active agent and an amino acid sequence capable of forming a reversible matrix formed of hydrogen bonds and/or hydrophobic interactions at the body temperature of a subject, wherein the amino acid sequence capable of forming the matrix at or around body temperature is a repeating peptide sequence comprising repeating units of from four to ten amino acids, and
one or more pharmaceutically acceptable excipients and/or diluents inducing the formation of the matrix upon administration,
wherein the formulation provides slow absorption from an injection site upon administration.

2. The pharmaceutical formulation of claim 1, wherein the amino acid sequence capable of forming the matrix at body temperature comprises [VPGXG]₉₀ where each X is selected from V, G, and A, and wherein the ratio of V:G:A may be about 5:3:2.

3. The pharmaceutical formulation of claim 2, wherein the amino acid sequence capable of forming the matrix at body temperature comprises [VPGXG]₁₂₀ where each X is selected from V, G, and A, and wherein the ratio of V:G:A may be about 5:3:2.

4. The pharmaceutical formulation of any one of claims 1 to 3, wherein the active agent is a protein.

5. The pharmaceutical formulation of claim 4, wherein the protein active agent has a circulatory half-life in the range of from about 30 seconds to about 10 hour, or about 30 seconds to about 1 hour.

6. The pharmaceutical formulation of claim 5, wherein the protein active agent is a GLP-1 receptor agonist or derivative thereof, a VPAC2 selective agonist or a derivative thereof, a GIP receptor agonist or a derivative thereof or a glucagon receptor agonist or a derivative thereof, or insulin or a derivative thereof.

7. The pharmaceutical formulation of any one of claims 1 to 6, wherein the therapeutic agent is selected from GLP-1 (A8G,7-37), ELP1-120, GLP-1 (A8G,7-37) ELP4-120 (PB1046), insulin EPL1-120, or VIP EPL1-120.

8. A sustained release pharmaceutical formulation comprising:
a therapeutic agent comprising an active agent and an amino acid sequence comprising [VPGXG]₉₀, where each X is selected from V, G, and A, and
one or more pharmaceutically acceptable excipients and/or diluents for formation of a reversible matrix from an aqueous form upon administration to a human subject, wherein the formulation provides slow absorption from an injection site upon administration.

9. The pharmaceutical formulation of claim 8, wherein the amino acid sequence comprises [VPGXG]₁₂₀, where each X is selected from V, G, and A, wherein V, G, and A are at a ratio of about 5:3:2.

10. The pharmaceutical formulation of any one of claims 8 to 9, wherein the active agent is a protein.

11. The pharmaceutical formulation of claim 10, wherein the protein active agent has a circulatory half-life in the range of from about 30 seconds to about 10 hours, or about 30 seconds to about 1 hour.

12. The pharmaceutical formulation of any one of claims 8 to 11, wherein the active agent is a GLP-1 receptor agonist or derivative thereof, a VPAC2 selective agonist or a derivative thereof, a GIP receptor agonist or a derivative thereof or a glucagon receptor agonist or a derivative thereof, or insulin or a derivative thereof.

13. The pharmaceutical formulation of any one of claims 8 to 12, wherein the active agent is selected from GLP-1 (A8G, 7-37) ELP1-120, GLP-1 (A8G,7-37) ELP4-120 (PB1046), insulin EPL1-120, and VIP ELP1-120.

14. A method for delivering a sustained release regimen of an active agent, comprising, administering the formulation of any one of claims 1 to 13, wherein the formulation is administered from about 1 to about 8 times per month.

15. The method of claim 14, wherein the active agent is GLP-1 or an analog thereof, or VIP or an analog thereof, or insulin or an analog thereof, and is administered about 1 to about 8 times per month.
